Europäisches Patentamt

European Patent Office

Office européen des brevets

(11) Numéro de publication: **0 008 554**
**B1**

(12)  **FASCICULE DE BREVET EUROPEEN**

(45) Date de publication du fascicule du brevet: **12.05.82**   (51) Int. Cl.³: **C 12 N 1/18, C 12 C 11/00**

(21) Numéro de dépôt: **79400555.3**

(22) Date de dépôt: **03.08.79**

(54) **Levure sèche active de panification; procédé et souche permettant de l'obtenir, application à la fermentation.**

(30) Priorité: **04.08.78 US 931151**

(43) Date de publication de la demande:
**05.03.80 Bulletin 80/5**

(45) Mention de la délivrance du brevet:
**12.05.82 Bulletin 82/19**

(84) Etats contractants désignés:
**DE GB IT NL SE**

(56) Documents cités:
**BE - A - 862 191**
**FR - A - 2 316 328**
**FR - A - 2 393 062**
**GB - A - 1 262 648**

Le dossier contient des informations techniques présentées postérieurement au dépôt de la demande et ne figurant pas dans le présent fascicule.

(73) Titulaire: **LESAFFRE et Cie**
**41, rue Etienne Marcel**
**F-75001 Paris (FR)**

(72) Inventeur: **Clement, Philippe**
**19, rue Payen**
**F-59100 Roubaix (FR)**
Inventeur: **Loiez, Annie**
**47, rue Roland**
**F-59000 Lille (FR)**

(74) Mandataire: **Koch, Gustave et al,**
**Cabinet PLASSERAUD 84, rue d'Amsterdam**
**F-75009 Paris (FR)**

Courier Press, Leamington Spa, England.

## 0 008 554

### Levure sèche active de panification; procédé et souche permettant de l'obtenir, application à la fermentation

L'invention a pour objet une nouvelle souche de levure de panification du genre de celles qui sont bien adaptées au maltose, osmotolérantes et résistantes au séchage. Elle vise également les levures actives sèches obtenues à partir de cette souche en tant que produit nouveau.

Des souches de levure du genre en question et les levures sèches correspondantes sont connues par le brevet français n° 77 39 149 du 23 décembre 1977.

Poursuivant ses travaux en vue de fournir à l'utilisateur des levures de panification de plus en plus performantes, la Société demanderesse, mettant en oeuvre le procédé d'obtention de nouvelles souches décrit dans le susdit brevet, a réussi à obtenir une nouvelle souche du genre en question qui est particulièrement avantageuse et qui fournit une levure sèche encore plus active que celles qui sont décrites dans ledit brevet.

Cette nouvelle souche selon l'invention, figurant dans les cahiers de laboratoire de la demanderesse sous le numéro a $\alpha$ 1217 a été déposée au National Collection of Yeast Cultures, Lyttel Hall, Nutfield, Redhill, Surrey RH1 4HY, Royaume Uni de Grande-Bretagne, sous le numéro de collection NCYC 890. La souche NCYC 890 a été également déposée sous le n°I-094 à la "Collection Nationale de microorganismes" tenue par l'Institut Pasteur, 128 rue du Docteur Roux, 75724 Paris Cédex 15 — France.

A l'aide de cette souche, on a préparé des levures fraîches (à environ 30% de matières sèches) rapides, bien adaptées au maltose et osmotolérantes. Grâce à sa résistance au séchage, on a pu préparer des levures sèches à plus de 92 pour cent de matières sèches conformes à l'invention.

La levure sèche conforme à l'invention est caractérisée par le fait que simultanément dans les tests A' décrits ci-après elle présente les caractéristiques suivantes:

— dans le test $A'_1$ sur 2 heures elle donne un dégagement gazeux compris entre 121 ml et 135 ml, et de préférence compris entre 125 ml et 135 ml.

— dans le test $A'_2$ sur 1 heure, elle donne un dégagement gazeux compris entre 53 ml et 60 ml et de préférence compris entre 55 ml et 60 ml.

— dans le test $A'_3$ sur 1 heure, elle donne un dégagement gazeux compris entre 47 ml et 54 ml.

— dans le test $A'_4$ sur 1 heure, elle donne un dégagement gazeux compris entre 26 ml et 30 ml.

Les tests A utilisés par la demanderesse pour caractériser les levures obtenues sont réalisés à l'aide du fermentomètre de Burrows et Harrison décrit dans le "Journal of the Institute of Brewing," Vol. LXV, No. 1, January-February 1959 et sont exactement définis de la manière suivante:

Test $A_1$ (levures comprimées fraîches)

— A 20 g de farine incubée à 30°C, on ajoute un poids de levure comprimée correspondant à 160 mg de matières sèches, cette levure étant délayée dans 15 ml d'eau contenant 27 g de NaCl par litre et 4 g de $SO_4 (NH_4)_2$ par litre; on malaxe à l'aide d'une spatule pendant 40 secondes, de manière à obtenir une pâte que l'on place an bain-marie réglé à 30°C; treize minutes après le début du malaxage, le récipient contenant le pâte est fermé hermétiquement; la quantité totale de gaz produit est mesurée après 60, puis 120 minutes; cette quantité est exprimée en ml à 30°C et sous 760 mm de Hg.

Test $A'_1$ (levures sèches)

— Identique à l'essai $A_1$, mais préalablement au malaxage, on réhydrate la levure sèche dans de l'eau distillée, à 38°C; on utilise à cet effet 40% du volume d'eau d'hydratation mis en oeuvre; le complément en eau, additionné de 405 mg de NaCl, est ajouté à l'issue des 15 minutes de réhydratation,

Test $A_2$ (levures comprimées fraîches)

— essai identique à l'essai $A_1$, mais on rajoute à la farine 100 mg de saccharose; la quantité totale de gaz produit est mesurée après 60 minutes,

Test $A'_2$ (levures sèches)

— essai identique à l'essai $A'_1$, mais on rejoute à la farine 100 mg de saccharose; la quantité totale de gaz produit est mesurée après 60 minutes,

Test $A_3$ (levures comprimées fraîches)

— essai identique à l'essai $A_1$, mais on rajoute à la farine 2 g de saccharose; la quantité totale de gaz produit est mesurée après 60 minutes,

Test $A'_3$ (levures sèches)

— essai identique à l'essai $A_1$, mais on rajoute à la farine 2 g de saccharose; la quantité totale de gaz produit est mesurée après 60 minutes,

Test $A_4$ (levures comprimées fraîches)

— essai identique à l'essai $A_1$, mais on rajoute à la farine 5,5 g de saccharose; la quantité totale

2

de gaz produit est mesurée après 60 minutes,

Test A'$_4$ (levures sèches)

— essai identique à l'essai A'$_1$, mais on rajoute à la farine 5,5 g de saccharose, la quantité totale de gaz produit est mesurée après 60 minutes.

La demanderesse a également utilisé comme test d'étude le test B$_1$ effectué sur zymotachygraphe CHOPIN (appareil vendu par les Ets TRIPETTE et RENAUD, 39 rue Jean-Jacques Rousseau, 75038 Paris Cédex 01), défini ci-après:

Test B$_1$ (levures comprimées fraîches et levures sèches instantanées n'ayant pas besoin d'une réhydratation préalable).

A 250 g de farine, on ajoute un poids de levure comprimée ou de levure sèche instantanée correspondant à 1,6 g de matières sèches de levure, et 150 ml d'eau salée (50 g de sel/1,5 l d'eau); on pétrit 6 minutes; la température de la pâte doit être de 27°C en fin de pétrissage; on place la pâte dans l'appareil et 6 minutes, exactement mesurées, après la fin du pétrissage, on met en pression la chambre thermostatée à 27°C; on mesure le dégagement total enregistré sur graphique, en ml, après 1 heure et 3 heures.

Dans ce test d'étude B$_1$ en 3 heures, la levure sèche selon l'invention donne en général un dégagement gazeux entre 1580 ml et 2000 ml.

Les valeurs caractéristiques de la levure sèche selon l'invention montrent qu'il s'agit d'une levure sèche qui est sensiblement plus rapide sur pâtes boulangères ayant jusqu'à 5% de sucre, que les meilleures levures sèches actuellement connues, cette levure sèche étant aussi performante sur pâtes boulangères ayant 5 à 15% de sucre que les meilleures levures sèches spécialisées actuellement connues. (Ces pourcentages sont calculés par rapport à la farine, en prenant le poids de sucre, du saccharose en général, ajouté à la farine).

Ces résultats sont la preuve de la bonne adaptation au maltose, de la bonne osmotolérance et de la bonne résistance au séchage de la souche conforme à l'invention.

Ceci étant, on a obtenu la souche conforme à l'invention de la manière suivante décrite ci-après:

Dans le but d'obtenir des nouveaux hybrides de levures osmotolérants (c'est-à-dire performants sur pâtes sucrées), résistants au séchage et significativement plus rapides et mieux adaptés au maltose qu'ceux obtenus jusqu'alors, on a procédé comme suit:

On a fait sporuler les meilleurs souches connues jusqu'alors comme combinant les propriétés d'adaptation au maltose, d'osmotolérance, de résistance au séchage, dont les exemples les plus représentatifs et les meilleurs sont les 5 souches déposées au National Collection of Yeast Cultures sous les numéros NCYC 847, NCYC 848, NCYC 878, NCYC 879 et NCYC 880.

On a croisé les haploïdes obtenus à partir de ces souches avec des haploïdes possédant la caractéristique d'une bonne adaptation au maltose, et ayant la propriété remarquable de conduire le plus souvent, après croisement, à des nouveaux hybrides possédant les propiétés recherchées. Des exemples représentatifs de ces haploïdes sont les haploïdes déposés au NCYC sous les numéros NCYC 881, NCYC 883, NCYC 884. La sporulation des souches de départ, l'obtention des haploïdes, la conjugaison de ces haploïdes sont effectuées selon les techniques décrites dans le chapitre 7 "Sporulation and Hybridization of Yeasts" écrit par R. R. FOWELL du livre "The Yeasts" Volume 1, édité par A. H. Rose and J. S. Harrison, 1969 — Academic Press, London and New York. La technique d'hybridation retenue a été la technique du mass-mating. Les tests de screening utilisés ont été les tests décrits dans le brevet français N° 77 39149. Les principaux tests de screening retenus dans ce travail ont été, le test appelé "premier test de screening" dans le susdit brevet (test qui consiste à mesurer le coefficient de multiplication moyen d'une souche donnée en suivant la variation de densité optique à 600 m$\mu$ d'une folie optique contenant 30 ml d'un milieu standard ensemencé par une suspension de la souche à tester, le critère de sélection utilisé étant que toute souche retenue à un coefficient de multiplication équivalent à celui des meilleures souches témoins de levure de panification), et le test appelé "troisième test de screening" (consistant à mesurer l'adaptation au maltose de ladite souche en présence de glucose, en déterminant la quantité de maltose restant dans un milieu standard ensemencé par la souche testée après une heure et après qu'une quantité de glucose ajoutée à ce milieu ait été complètement consommée), le critère de sélection utilisé dans ce "troisième test" étant que toute souche retenue à une adaptation au maltose correspondant à une quantité de maltose consommée égale à au moins 90% du maltose consommé dans le test par les meilleures souches témoins adaptées au maltose. Les autres tests de screening décrits dans le brevet français susmentionné, principalement le "quatrième test" (recherche d'une invertase relativement basse, rejet de toutes les souches présentant plus de 45 unités invertase. L'unité invertase étant définie comme la production d'une micromole de sucres réducteurs en 5 minutes par mg de matières sèches de levures, à 30°C et à pH 4,7, sans plasmolyse de la levure, soit une demi-micromole de saccharose interverti), n'ont été effectués que sur les hybrides qui se sont révélés les plus performants à l'issue des tests de screening décrits ci-dessus (appelés premier et troisième tests).

Ce travail d'hybridation et de sélection a conduit à sélectionner la souche hybride conforme à

**0 008 554**

l'invention, désignée dans les cahiers de laboratoire de la demanderesse sous l'appellation a α 1217.

Un premier envoi de cette nouvelle souche, correspondant à la souche diploïde obtenue par mass-mating a été envoyé au NCYC le 30 juin 1978 et a reçu le numéro NCYC 889. L'isolement purifié correspondant à cette obtention a été déposé à la "National Collection of Yeast Cultures" sous le n° NCYC 890 le 18 juillet 1978. La "National Collection of Yeast Cultures" a envoyé cette souche NCYC 890 le 11 juillet 1979 à la "Collection Nationale de Microorganismes" tenue par l'Institut Pasteur de Paris qui l'a reçue le 19 juillet et lui a donné le numéro I-094.

Cette nouvelle souche a α 1217 appartient à l'espèce Saccharomyces Cerevisiae, comme ses deux souches parentes. En culture en milieu liquide de marque DIFCO YM-Broth, elle donne, après 24 heures ou 72 heures, des cellules rondesovales ou ovales, le plus souvent seules, ou en paires de dimensions en microns (3—5) × (4—8) au bout de 24 heures, et (2,5—5) × (4—8) au bout de 72 heures. Elle ne sporule pas très facilement, il y a 1 à 4 ascospores par asque; il y a en général peu d'asques à 4 spores. Elle fermente le dextrose, le maltose, le saccharose, le raffinose, difficilement le galactose. Elle assimile, en plus des sucres déjà cités, le tréhalose, le mélézitose, l'acide lactique, le D-mannitol et 1'α-méthylglucoside. Cette liste n'est pas exhaustive. La plupart de ces caractéres détermines dans les tests de Lodder, sont des caractéres secondaires, sans signification technologique. Leur reproductibilité, dans le cadre des tests pratiqués, est mauvaise, elle dépend des impuretés que peuvent contenir certains sucres, de l'état physiologique des cultures au moment des tests.

Cette souche a été cultivée sur fermenteurs de 3 litres tels que décrits dans "Yeast Technology," J. White (1954), pages 103 à 106, où le milieu de culture a un volume total de 1100 ml, le sucre est apporté sous forme de mélasse, l'air est filtré sur membrane du type Millipore à raison de 1 m³/heure et l'ensemencement est réalisé par 300 mg de levure obtenue par culture anaérobie en ballons.

| Souche purifiée | invertase | Test A₁ 1 heure | Test A₂ 1 heure | Test A₃ 1 heure | Test A₄ 1 heure |
|---|---|---|---|---|---|
| Essai 1 |  | 52,6 | 61 | 56 | 23,5 |
| Essai 2 | 41 | 57,2 | 64,9 | 56,4 | 19,6 |
| Essai 3 | 38 | 56,2 | 64,3 | 55,8 | 21 |
| Essai 4 | 41 | 56,8 | 65,1 | 56,6 | 20,3 |
| Essai 5 |  | 54,9 | 61,8 | 52,9 | 19,4 |

Cette première série de cultures permet de voir que cette souche donne des résultats excellents dans les tests A₁, A₂ et A₃, un résultat moyen dans le test A₄.

Cette souche a également été testée sur batterie de fermenteurs NBS (New Brunswick Scientific Co., Inc.) de volume total 5 litres et de volume utile 3 à 3,5 litres, permettant une alimentation continue en les différents nutriments de la levure (mélasse, azote, $P_2O_5$). Ces essais sont conduits selon les règles habituelles en matière de propagation des levures telles que décrites, par exemple dans l'ouvrage "Yeast Technology" de Gerald Reed and Henry J. Peppler (1973), The Avi Publishing Company Inc. Tous les nutriments nécessaires en petites quantités à la levure, comme par exemple le magnésium ou les facteurs de croissance habituellement employés (biotine, vitamines du groupe B), sont apportés en début de culture. La multiplication de la levure est de l'ordre de 4 à 5 en 10 heures. Un premier essai a donné les résultats suivants mesurés sur la levure fraîche obtenue à environ 30% de matières sèches.

| Azote sur matières sèches de la levure obtenue | Test A₁ en 2 heures | Test A₂ en 1 heure | Test A₃ en 1 heure | Test A₄ en 1 heure |
|---|---|---|---|---|
| 7,2 | 134 | 63 | 53 | 23 |

Une deuxième série de 7 essais a donné en moyenne les résultats suivants mesurés sur la levure fraîche récoltée.

| Azote sur matières sèches | Test A₁ | Test A₄ |
|---|---|---|
| 7,80 | 144,4 | 23,25 |

Ces essais confirment les essais précédents sur fermenteurs type White indiquant que cette souche donne d'excellents résultats dans les tests $A_1$, $A_2$ et $A_3$, un résultat moyen dans le test $A_4$. Les résultats positifs de tous ces essais au stade 3 litres de volume utile ont permis de passer directement à des essais au stade usine.

A l'exception de l'emploi des procédés et matériels spécifiés ci-après, la souche a été propagée en usine en plusieurs stades de multiplication, la levure fraîche a été récoltée à l'aide de matériels classiques employés en levurerie et selon des procédés de fabrication usuels, comme les matériels et les procédés qui sont décrits par exemple dans le livre "Yeast Technology," 1973, déjà cité.

Le dernier stade de multiplication de la levure conduisant à la levure récoltée en vue d'être séchée a été mené de manière à obtenir une levure stable au séchage en respectant la règle suivante:

Le culture de la souche est conduite de manière à obtenir une levure fraîche à 30—35% de matières sèches présentant:

. une quantité de bourgeons inférieure à 5% et, de préférence, inférieure à 1%,

. une teneur en protéines correspondant à l'optimum d'activité de la souche cultivée, compte tenu de la perte d'activité au séchage,

. une composition répondant aux inégalités suivantes:

$$\frac{\text{tréhalose}}{\text{matiérès sèches}} \geqslant 12\ \%$$

$$2,3 \leqslant \frac{\text{azote}}{P_2O_5} \leqslant 3,8$$

. un abaissement cryoscopique de l'eau externe de la levure inférieur à 0,5°C et, de préférence, inférieur à 0,3°C (cet abaissement cryoscopique étant mesuré de la manière suivante : on réalise une crème avec 100 g de la levure fraîche pressée sur laquelle on veut effectuer cette mesure et 30 g d'eau déminéralisée, on centrifuge cette crème et on mesure l'abaissement cryoscopique du surnageant obtenu).

Cette souche a la particularité de rester stable au séchage malgré des teneurs en azote sur matières sèches atteignant ou dépassant 8,5%.

On donne dans les tableaux ci-après, les résultats observés avec cette nouvelle souche a $\alpha$ 1217 après culture d'environ 100 m³ conduisant à récolter environ 25 tonnes de levure fraîche, menée de manière à obtenir:

. une teneur en azote sur matières sèches d'environ 8% (généralement comprise entre 7,6% et 8,3%),

. une teneur en $P_2O_5$ sur matières sèches d'environ 2,3%,

. une teneur en tréhalose sur matières sèches d'environ 13%,

. un taux de bourgeons de l'ordre de 1%,

. un abaissement cryoscopique de l'eau externe de la levure de l'ordre de 0,3°C.

| Levure fraîche à environ 30 % de matières sèches | | | |
|---|---|---|---|
| Test A₁ 2 heures | Test A₂ 1 heure | Test A₃ 1 heure | Test A₄ 1 heure |
| 145 | 65 | 58 | 33 |

A cette levure fraîche stable au séchage, obtenue de préférence entre 30 et 35% de matières sèches, on ajoute une fine émulsion constituée d'un émulsifiant, comme les esters de sorbitol ou de polyglycérol à raison de 1 à 2% des matières sèches levure et éventuellement un agent épaississant. On l'extrude à travers une grille de largeur de maille 0,5 mm à 2 mm, de préférence 0,5 mm à 1 mm et on la sèche à au moins 92% de matières sèches par un séchage particulièrement ménageant, c'est-à-dire un séchage relativement court de moins de 4 heures et où la température de la levure ne dépasse pas 30°C en début de séchage et 40°C en fin de séchage.

A la levure fraîche stable au séchage, obtenue avec la souche a $\alpha$ 1217, on a ajouté une fine émulsion constituée d'ester de sorbitol et de gomme arabique à raison respectivement de 1,5% et de 0,8% de la matière sèche levure. On l'a extrudée en vermicelles de 0,6 mm de diamètre. On l'a séchée en laboratoire sur un fluidiseur discontinue de laboratoire. On a porté une attention particulière à ce que le début de séchage soit rapide et homogène. La durée totale du séchage a été inférieure à 1 heure, la température de la levure a été maintenue en dessous de 30°C en début de séchage et en dessous de 35°C en fin de séchage. Dans ces essais, la levure sèche a été portée à plus de 94% de matières sèches pour obtenir une bonne conservation de ses propriétés.

| | Levures sèches à environ 95 % de matières sèches | | | | |
|---|---|---|---|---|---|
| | Test $A_1'$ 1h + 1h = 2h | Test $A_2'$ 1 heure | Test $A_3'$ 1 heure | Test $A_4'$ 1 heure | Test $B_1$ 3 heures |
| Essai 1 | 53 + 71 = 124 | 57 | 49 | 27 | |
| Essai 2 | 56 + 74 = 130 | 58 | 50 | 27 | |
| Essai 3 | 52 + 70 = 122 | 53 | 48 | 29 | 1580 |
| Essai 4 | 53 + 70 = 123 | 56 | 49 | 26 | 1670 |
| Essai 6 | 55 + 73 = 128 | 58 | 51 | 28 | |
| Essai 7 | 58 + 76 = 134 | 59 | 53 | 26,5 | |
| Essai 8 | 57 + 75 = 132 | 59 | 53 | 27 | |

Ces résultats montrent que la levure fraîche obtenue avec la souche a $\alpha$ 1217 est un peu plus rapide que celles qui peuvent être obtenues avec les meilleures souches connues. Ces écarts sont nettement creusés en ce qui concerne les levures sèches que cette souche permet d'obtenir. Ces résultats méritent d'être comparés avec ceux des levures sèches obtenues dans les mêmes conditions d'essais avec les souches connues jusqu'alors comme donnant les meilleures levures sèches commerciales dans chacun des tests $A_1'$, $A_2'$, $A_3'$, $A_4'$.

| Souches | Levures sèches à environ 95 % de matières sèches | | | | |
|---|---|---|---|---|---|
| | $A'_1$ 1h + 1h = 2h | $A'_2$ 1 heure | $A'_3$ 1 heure | $A'_4$ 1 heure | $B_1$ 3 heures |
| Hybride de levure repide adapté au maltose | 48 + 70 = 118 | 50 | 41 | 18 | 1500 |
| NCYC R30 | 32 + 43 = 75 | 45 | 47 | 32 | 1050 |
| NCYC 848 | 51 + 68 = 119 | 53 | 45 | 25 | 1550 |
| NCYC 847 | 46 + 61 = 107 | 53 | 48 | 28 | 1470 |
| NCYC 878 | 37 + 62 = 99 | 47 | 48 | 35 | |
| Souche a $\alpha$ 1217 NCYC 890 | 55 + 73 = 128 | 57 | 50 | 27,5 | 1700 |

On voit ainsi que la souche a $\alpha$ 1217 permet d'obtenir des levures sèches plus rapides que toutes les levures sèches obtenues auparavant dans les tests $A'_1$, $A'_2$, $A'_3$ qui correspondent aux conditions de fermentation rencontrées dans les pâtes non sucrées, peu sucrées ou moyennement sucrées (c'est-à-dire contenant jusqu'à environ 5% de saccharose ajouté à la farine). Dans le test $A'_1$, cette souche permet de dépasser systématiquement en 2 heures le chiffre de 120 ml de $CO_2$ qui n'était qu'extrêmement rarement atteint ou dépassé (et ceci de très peu) auparavant. Dans le test $A'_4$, correspondant aux conditions de fermentation rencontrées dans des pâtes contenant plus de 15% de saccharose ajouté à la farine, elle donne un résultat 50% supérieur à celui obtenu avec un hybride de levure rapide adapté au maltose, mais cependant encore légèrement inférieur à celui obtenu avec les meilleures souches très osmotolérantes. Cet ensemble de caractères, plus grande activité sur pâtes jusqu'à environ 5% de sucre ajouté à la farine, activité équivalente aux meilleures levures sèches connues dans un intervalle 5 à 15% de sucre adjouté à la farine, performances acceptables au-delà de 15% de sucre ajouté à la farine, en font une nouvelle levure sèche aux caractéristiques très intéressantes.

L'invention vise également l'emploi des nouvelles levures obtenues avec la souche a $\alpha$ 1217 et, en particulier, l'emploi des nouvelles levures sèches conformes à l'invention dans les procédés de panification.

La souche a $\alpha$ 1217 a la propriété de bien fermenter dans des milieux complexes riches en sucre, c'est une souche particulièrement rustique s'adaptant bien à toutes les conditions des milieux de fermentation. Cette souche, en conséquence, donne une très bonne levure de distillerie.

L'invention vise également un procédé de production de biomasse de levure permettant de produire des levures fraîches ou sèches pour la panification, mais aussi la distillerie, et la fermentation de tous les jus sucrés, en particulier de ceux conduisant à l'obtention de boissons alcoolisées.

**Revendications**

1. Levure sèche active de panification à teneur en matières sèches supérieure à 94% et contenant un émulsifiant, caractérisée par le fait que simultanément:
— dans le test $A'_1$, sur 2 heures, elle donne un dégagement gazeux compris entre 125 ml et 135 ml,
— dans le test $A'_2$, sur 1 heure, elle donne un dégagement gazeux compris entre 55 ml et 60 ml,
— dans le test $A'_3$, sur 1 heure, elle donne un dégagement gazeux compris entre 47 ml et 54 ml,
— dans le test $A'_4$, sur 1 heure, elle donne un dégagement gazeux compris entre 26 ml et 30 ml.

2. Levure sèche active de panification selon la revendication 1, caractérisée par le fait qu'elle a été obtenue à partir de la souche a $\alpha$ 1217 ayant fait l'objet d'un dépôt au National Collection of Yeast Cultures sous le n° NCYC 890 et à la Collection Nationale de microorganismes tenue par l'Institut Pasteur sous le n° I-094.

3. Application à la fermentation des jus sucrés rencontrés en distillerie et dans la fabrication de boissons alcoolisées de la nouvelle levure sèche active de panification, selon l'une des revendications 1 et 2.

**0 008 554**

4. Procédé de préparation d'une levure fraîche comprimée à environ 30% de matières sèches et de la levure sèche active à au moins 94% de matières sèches, obtenue à partir de cette levure fraîche, l'une et l'autre étant destinée à la fermentation panaire, à la fermentation des jus sucrés rencontrés en distillerie et dans la fabrication de boissons alcoolisées, caractérisé par le fait que l'on cultive la souche a $\alpha$ 1217.

5. Souche de levure a $\alpha$ 1217 ayant fait l'objet d'un dépôt au National Collection of Yeast Cultures sous le n° NCYC 890 et à la Collection Nationale de microorganismes tenue par l'Institut Pasteur sous le n° I-094, et propre à permettre l'obtention d'une levure destinée à la fermentation panaire, à la fermentation des jus sucrés, notamment d'une levure sèche active de panification donnant simultanément dans les tests $A'_1$ à $A'_4$ des valeurs situées à l'intérieur des domaines suivants:

— test $A'_1$ : sur 2 heures, dégagement gazeux compris entre 125 ml et 135 ml,
— test $A'_2$ : sur 1 heure, dégagement gazeux compris entre 55 ml et 60 ml,
— test $A'_3$ : sur 1 heure, dégagement gazeux compris entre 47 ml et 54 ml,
— test $A'_4$ : sur 1 heure, dégagement gazeux compris entre 26 ml et 30 ml.

## Claims

1. Active dry baker's yeast having a dry matter content higher than 94% and containing an emulsifying agent, characterized by the fact that simultaneously:

— in test $A'_1$, in two hours, it gives a gas release comprised between 125 ml and 135 ml,
— in test $A'_2$, in one hour, it gives a gas release comprised between 55 and 60 ml,
— in test $A'_3$, in one hour, it gives a gas release comprised between 47 ml and 54 ml,
— in test $A'_4$, in one hour, it gives a gas release comprised between 26 ml and 30 ml.

2. Active dry baker's yeast according to Claim 1, characterized by the fact that it was obtained from the strain a $\alpha$ 1217 deposited at the National Collection of Yeast Cultures under the number NCYC 890 and at the Collection Nationale de microorganismes of Institut Pasteur under the number I-094.

3. Use for the fermentation of sugar juices occurring in distillery and in the manufacture of alcoholic beverages of the new dry active baker's yeast according to one of Claims 1 and 2.

4. Process for the preparation of a fresh compressed yeast having a dry matter content of about 30% and of the dry active yeast having a dry matter content of at least 94%, obtained from said fresh yeast, the one and the other being intended to the fermentation occurring in the making of bread, to the fermentation of sugar juices occurring in distillery and in the manufacture of alcoholic beverages, characterized by the fact that the strain a $\alpha$ 1217 is cultivated.

5. Yeast strain a $\alpha$ 1217 deposited at the National Collection of Yeast Cultures under the number NCYC 890 and at the Collection Nationale de microorganismes of Institut Pasteur under the number I-094, and proper to enable the obtaining of a yeast intended to the fermentation occurring in the making of bread, to the fermentation of sugar juices, in particular proper to enable the obtaining of an active dry baker's yeast giving simultaneously in the tests $A'_1$ to $A'_4$ the values situated within the following ranges:

—Test $A'_1$ : in two hours, gas release comprised between 125 and 135 ml,
—Test $A'_2$ : in one hour, gas release comprised between 55 and 60 ml,
—Test $A'_3$ : in one hour, gas release comprised between 47 and 54 ml,
—Test $A'_4$ : in one hour, gas release comprised between 26 and 30 ml.

## Patentansprüche

1. Aktive Trockenbäckerhefe mit einem Trockensubstanzgehalt von über 94% und enthaltend ein Emulgiermittel, dadurch gekennzeichnet, dass sie gleichzeitig:

— im Test $A'_1$, in zwei Stunden, zu einer zwischen 125 ml und 135 ml liegenden Gasfreisetzung führt,
— im Test $A'_2$, in einer Stunde, zu einer zwischen 55 ml und 60 ml liegenden Gasfreisetzung führt,
— im Test $A'_3$, in einer Stunde, zu einer zwischen 47 ml und 54 ml liegenden Gasfreisetzung führt,
— im Test $A'_4$, in einer Stunde, zu einer zwischen 26 ml und 30 ml liegenden Gasfreisetzung führt.

2. Aktive Trockenbäckerhefe nach Anspruch 1, dadurch gekennzeichnet, dass sie ausgehend von Stamm a $\alpha$ 1217 erhalten wurde, wobei dieser Stamm bei der National Collection of Yeast Cultures unter der Nummer NCYC 890 und bei der Collection Nationale de Microorganismes im Institut Pasteur unter der Nummer I-094 hinterlegt ist.

3. Anwendung der aktiven Trockenbäckerhefe nach einem der Ansprüche 1 und 2 bei der Gärung von gezuckerten Säften, wie sie im Destilleriewesen, sowie bei der Herstellung von alkoholischen Getränken angetroffen werden.

4. Verfahren zur Herstellung einer frischen Presshefe mit etwa 30% Trockensubstanz, sowie der daraus erhaltenen Trockenhefe mit zumindest 94% Trockensubstanz, wobei beide Hefen für die Gärung die bei der Brotherstellung eintritt, sowie für die Gärung der im Destilleriewesen und bei der Herstellung von alkoholischen Getränken angetroffenen gezuckerten Säften, bestimmt sind, dadurch gekennzeichnet, dass man dem Stamm a $\alpha$ 1217 züchtet.

5. Hefestamm a $\alpha$ 1217 hinterlegt bei der National Collection of Yeast Cultures unter der Nummer NCYC 890, sowie bei der Collection Nationale de Microorganismes im Institut Pasteur unter der Nummer I-094 und geeignet zum Erhalten einer Hefe, die für die Gärung bei der Brotherstellung und für die Gärung von gezuckerten Säften bestimmt ist, insbesondere einer aktiven Trockenbäckerhefe, die gleichzeitig in den Testen $A'_1$ bis $A'_4$ zu Werten führt, die innerhalb folgender Bereiche liegen:

— Test $A'_1$ : in zwei Stunden, Gasfreisetzung zwischen 125 ml und 135 ml,
— Test $A'_2$ : in einer Stunde, Gasfreisetzung zwischen 55 ml und 60 ml,
— Test $A'_3$ : in einer Stunde, Gasfreisetzung zwischen 47 ml und 54 ml,
— Test $A'_4$ : in einer Stunde, Gasfreisetzung zwischen 26 ml und 30 ml.